# EUROPEAN PATENT APPLICATION

(11) **EP 3 005 929 A1**
(43) Date of publication of application: **13.04.2016**
(21) Application number: 14801815.3
(22) Date of filing: 09.05.2014
(51) Int. Cl.: A61B 1/00, G02B 23/24

(54) **TUBULAR SYSTEM**

(30) Priority: 24.05.2013 JP 2013109845
(71) Applicant: Olympus Corporation, Shibuya-ku Tokyo 151-0072 (JP)
(72) Inventor: FUJITA, Hiromasa, Shibuya-ku Tokyo 151-0072 (JP); ITO, Takeshi, Shibuya-ku Tokyo 151-0072 (JP)
(74) Representative: Winter, Brandl, Fürniss, Hübner, Röss, Kaiser, Polte - Partnerschaft mbB
(86) International application number: PCT/JP2014/062504
(87) International publication number: WO 2014/188890

(57) **Abstract**

A tubular system includes a tubular apparatus 100 including a flexible inserting section and a physical quantity detector 111, a physical quantity detector signal processing box 300 to process a signal from the physical quantity detector, and a detachable connection cable 200 to connect the tubular apparatus 100 with the physical quantity detector signal processing box300. The connection cable 200 includes a light source 211 to supply light to an optical waveguide of the physical quantity detector 111, a photodetector 214 to detect light modulated by the physical quantity detector 111, a light entrance/exit section to supply or receive an optical signal to or from the physical quantity detector 111, and an electrical input/output section to supply or receive an electrical signal to or from the physical quantity detector signal processing box.

## Description

### Technical Field

The present invention relates to a tubular system including a tubular apparatus having a flexible inserting section.

### Background Art

As tubular apparatuses each having a flexible inserting section, there are endoscopes. Japanese Patent No. 4694062 discloses one of such endoscopes. This endoscope is provided with a function of detecting a bend of an inserting section flexible tube, and hence a flexible strip-like member having bend detection optical fibers arranged therein is arranged in the inserting section flexible tube. In each bend detection optical fiber, a bend detecting section whose light transmission amount varies in accordance with a degree of bending is formed. Further, in a connector, there are arranged a light-emitting to cause light to enter the bend detection optical fibers, a photodiode to photoelectrically convert light exiting from the bend detection optical fibers to output an electrical signal, and others.

### Summary of Invention

Such an endoscope provided with a light-emitting diode and a photodiode as in the above constitution has the following problems.

The endoscope must be subjected to sterilization with a high-temperature/high-pressure/corrosive gas after use. Thus, the endoscope is required to be configured to protect the light-emitting diode and the photodiode from the sterilization. This will be causes of cost increase and size enlargement.

Furthermore, in the tubular system, it is often performed that the tubular apparatus such as an endoscope is replaced and used. That is, one tubular apparatus processing box (e.g., an endoscope image processing circuit or an endoscope illumination box) is often shared with tubular apparatuses. In each of all the endoscopes, the light-emitting diode and the photodiode are required to be mounted therein. This will be causes of cost increase of the endoscope and limitation of versatility of the tubular apparatus processing box.

In view of the above-described actual situation, an object of the present invention is to provide a tubular system including a tubular apparatus configured to be advantageous to production or a size and also has the high versatility.

A tubular system according to the present invention includes a tubular apparatus including a flexible inserting section and a physical quantity detector configured to detect a physical quantity, a physical quantity detector signal processing box to process a signal from the physical quantity detector, and a detachable connection cable to connect the tubular apparatus with the physical quantity detector signal processing box. The physical quantity detector includes an optical waveguide arranged in the inserting section. The connection cable includes a light source to supply light to the optical waveguide of the physical quantity detector, a photodetector to detect light modulated by the physical quantity detector, a light entrance/exit section to supply or receive an optical signal to or from the physical quantity detector, and an electrical input/output section to supply or receive an electrical signal to or from the physical quantity detector signal processing box. The physical quantity detector, the light source, the photodetector, and the light entrance/exit section constitute a sensor to detect the physical quantity.

According to the present invention, the tubular system including the tubular apparatus configured to be advantageous to production or a size and also has the high versatility is provided.

### Brief Description of Drawings

FIG. 1 shows a structural example of a tubular system;
FIG. 2 shows another structural example of the tubular system;
FIG. 3 shows still another structural example of the tubular system;
FIG. 4 shows a structural example of a photodetector;
FIG. 5 shows a connection example of the tubular system;
FIG. 6 shows a structural example of a connection cable;
FIG. 7 shows another structural example of the connection cable;
FIG. 8 shows a connection example of the tubular system including the connection cable in FIG. 7;
FIG. 9 shows still another structural example of the connection cable;
FIG. 10 shows a connection example of the tubular system including the connection cable in FIG. 9;
FIG. 11 shows emission spectrums of a light source constituted of two light-emitting elements;
FIG. 12 shows a connection example of a tubular apparatus and the connection cable;
FIG. 13 shows another connection example of the tubular apparatus and the connection cable;
FIG. 14 shows still another connection example of the tubular apparatus and the connection cable;
FIG. 15 shows yet another connection example of the tubular apparatus and the connection cable;
FIG. 16 shows still yet another connection example of the tubular apparatus and the connection cable;
FIG. 17 is schematic views of light transmission amounts according to curving of an optical fiber;
FIG. 18 shows a structural example of a fiber sensor that is a temperature sensor; and
FIG. 19 shows still another example of the fiber sensor that is the temperature sensor;

### Description of Embodiments

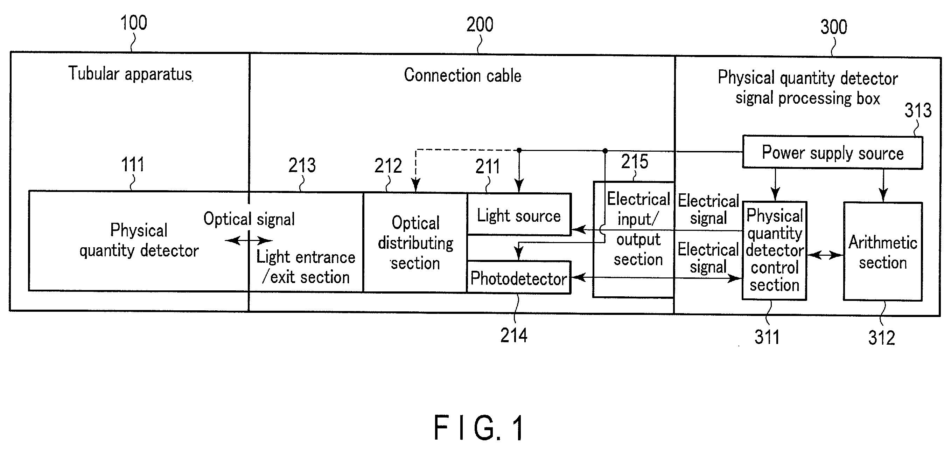

Embodiments will now be described hereinafter with reference to the drawings. FIG. 1 shows a structural example of a tubular system. As shown in FIG. 1, the tubular system includes a tubular apparatus 100 including a flexible inserting section to be inserted into a tube space of a specimen and a physical quantity detector 111 configured to detect a physical quantity, a physical quantity detector signal processing box 300 to process signals from the physical quantity detector 111, and a detachable connection cable 200 to connect the tubular apparatus 100 with the physical quantity detector signal processing box 300.

The tubular apparatus 100 may be a medical endoscope, an industrial endoscope, a catheter, or the like. Physical quantities detected by the physical quantity detector 111 are a shape, temperatures, a position, and others of the inserting section of the tubular apparatus 100. The physical quantity detector 111 includes an optical waveguide arranged in the inserting section of the tubular apparatus 100. The optical waveguide may be constituted of, e.g., an optical fiber. Alternatively, the optical waveguide may be constituted of a membranous waveguide having a flexible laminated structure through which light is confined and transmitted and that has flexibility in a detection obj ect section 132. Light propagated through the optical waveguide is modulated in dependence on a shape, temperatures, a position, and others of the inserting section of the tubular apparatus 100.

The connection cable 200 includes a light source 211 to supply light to the optical waveguide of the physical quantity detector 111, a photodetector 214 to detect light modulated by the physical quantity detector 111, a light entrance/exit section 213 to supply and receive optical signals to or from the physical quantity detector 111, and an electrical input/output section 215 to supply and receive electrical signals to or from the physical quantity detector signal processing box 300. The connection cable 200 may have an optical distributing section 212 defining flows of light between the light entrance/exit section 213, the light source 211, and the photodetector 214. Although not shown, the connection cable 200 may include a feedback system to stably drive the light source 211 mounted therein.

The physical quantity detector 111 of the tubular apparatus 100 cooperates with the light source 211, the photodetector 214, and the light entrance/exit section 213 in the connection cable 200 to constitute a sensor to detect the physical quantities. This sensor may be constituted of, e.g., a sensor to detect a change in light quantity of light (e.g., a fiber sensor or a long-period FBG sensor) or a sensor to detect a change in wavelength of light (e.g., a short-period FBG sensor). Besides, it is possible to adopt any sensor as long as it is a sensor using the optical waveguide and having a system to supply light to the optical waveguide and detect light propagated through the optical waveguide.

The physical quantity detector signal processing box 300 has a physical quantity detector control section 311 to control the physical quantity detector 111, an arithmetic section 312 to calculate a physical quantity of the inserting section of the tubular apparatus 100, and a power supply source 313 to supply electric power to the light source 211, the optical distributing section 212, the photodetector 214, the physical quantity detector control section 311, and the arithmetic section 312. The physical quantity detector signal processing box 300 may be constituted of, e.g., a computer (PC), and it may be integrally constituted with a tubular apparatus image processing box or a tubular apparatus light source box.

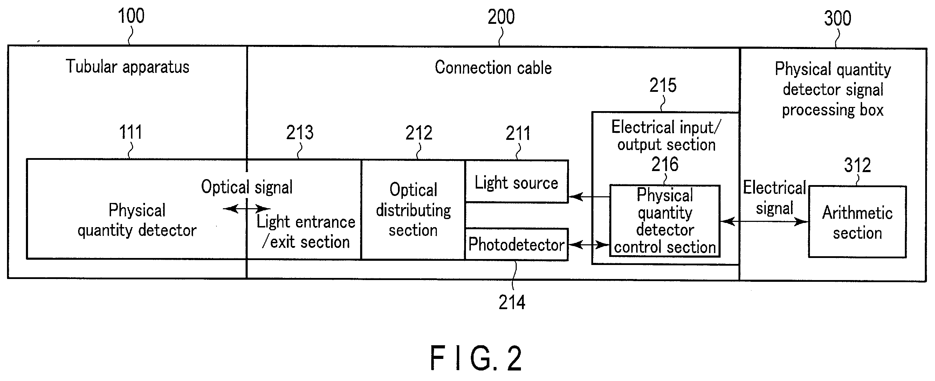

FIG. 2 shows another structural example of the tubular system. Like reference numerals denote members equal to those depicted in FIG. 1 to omit a detailed description thereof. As shown in FIG. 2, in this tubular system, the physical quantity detector signal processing box 300 does not have the physical quantity detector control section 311, but the electrical input/output section 215 of the connection cable 200 has a physical quantity detector control section 216 to control the physical quantity detector 111 instead. Although not shown, the connection cable 200 may include a power supply source to supply electric power to the light source 211, the optical distributing section 212, the photodetector 214, and the physical quantity detector control section 216. Moreover, the physical quantity detector signal processing box 300 does not have the arithmetic section 312, but the electrical input/output section 215 of the connection cable 200 may have the arithmetic section 312 instead.

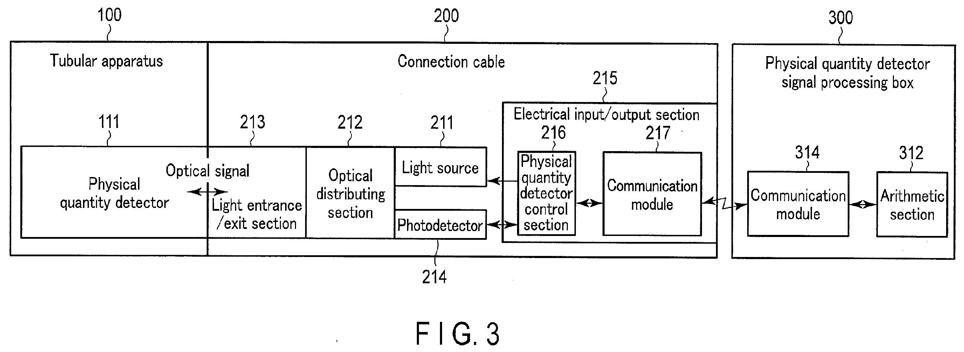

FIG. 3 shows still another example of the tubular system. Like reference numerals denote members equal to the members shown in FIG. 2 to omit a detailed description thereof. As shown in FIG. 3, in this tubular system, the electrical input/output section 215 of the connection cable 200 includes a communication module 217 in addition to the physical quantity detector control section 216, the physical quantity detection signal processing box 300 includes a communication module 314, and electrical signals are wirelessly transmitted or received between the electrical input/output section 215 and the physical quantity detection signal processing box 300.

In such a tubular system, even if the tubular apparatus 100 is exposed to a sterilization environment using a high-temperature/high-pressure/corrosive gas, since components that may be possibly deteriorated or corroded, e. g. , the light source 211, the photodetector 214, and the optical distributing section 212, are not mounted in the tubular apparatus 100, a possibility of a failure or a deterioration of performance can be greatly suppressed. At this time, since constituent sections concerning light, e.g., the light source 211, the photodetector 214, the light entrance/exit section 213, and the optical distributing section 212, are arranged in the connection cable 200, so that a connecting position of optical signals is restricted to one position between the tubular apparatus 100 (a medical endoscope, an industrial endoscope, or a catheter) and the connection cable 200, signal connection loss hardly occurs, and a tubular system that is easy to handle can be provided. Furthermore, if a medical institution or the like prepares the tubular apparatus 100 in advance, more tubular apparatus 100 than the tubular apparatus processing boxes (e.g., the endoscope image processing circuits or the endoscope illumination boxes) of the tubular apparatuses 100 are prepared. Under such circumstances, the number of expensive components (e.g., the light source 211, the photodetector 214, the light entrance/exit section 213, the optical distributing section 212, and the like) could be reduced, and these components substantially equal to the tubular apparatus processing boxes in number could be prepared. If the light source 211, the photodetector 214, the light entrance/exit section 213, and the optical distributing section 212 as well are mounted in the connection cable 200, an effect of reducing costs can be provided.

Although providing the tubular apparatus processing box with a function of the connection cable 200 and a function of the physical quantity detector signal processing box 300 can be considered, the versatility (enabling connecting any type of tubular apparatus 100 as much as possible, enabling connecting even if a type of physical quantity detector 111 is different) and extensibility of the tubular apparatus processing box are deteriorated in this case. Contrarily, in the tubular system according to this embodiment, since the connecting cable 200 and the physical quantity detector signal processing box 300 are independent from the tubular apparatus processing box, the versatility and extensibility of the tubular apparatus processing box is excellent.

When the physical quantity detector control section 216, the arithmetic section 312, and the communication module 217 as well are subjected to miniaturization/high-density packaging in the connection cable 200 and an output from the physical quantity detector 111 is converted into a highly versatile data format and then output, the connection cable 200 can be connected to a versatile data communication connector of the tubular apparatus processing box or a communication (wireless communication, RS-232C, USB, or the like) connector of a computer (PC) and then used, and both the tubular apparatus 100 and the physical quantity detector 111 have configurations with the high versatility.

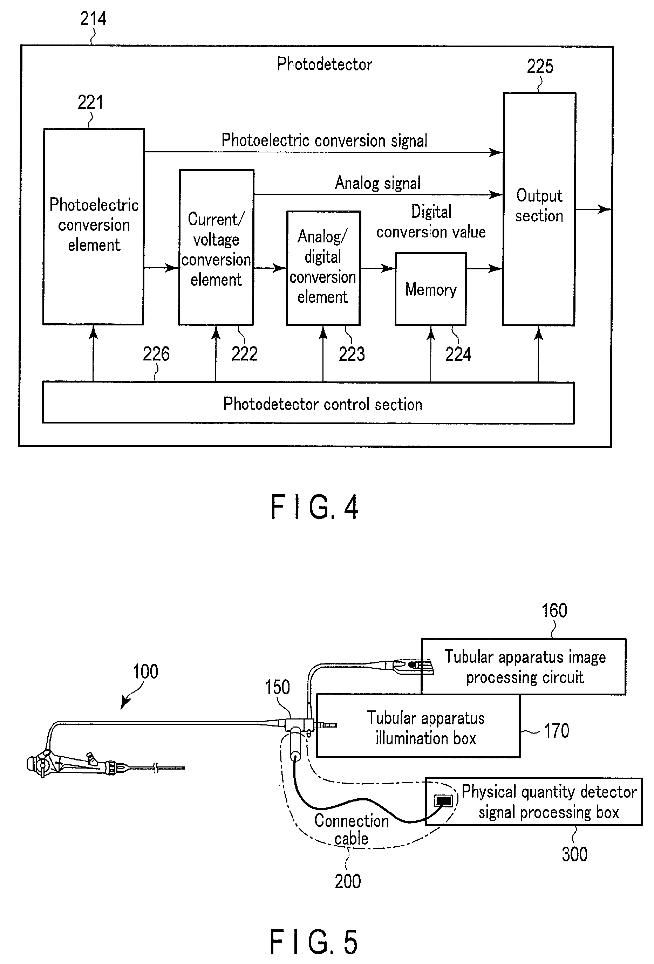

FIG. 4 shows a structural example of the photodetector 214. As shown in FIG. 4, the photodetector 214 includes a photoelectric conversion element 221 to convert an optical signal modulated by the physical quantity detector 111 into an electrical signal. The photodetector 214 may additionally include a current/voltage conversion element 222 to convert a current signal after the photoelectric conversion into a voltage signal, an analog/digital conversion element 223 to convert an analog signal after the current/voltage conversion into a digital signal, and a memory 224 to store a digital conversion value after the analog/digital conversion. The photodetector 214 also includes an output section 225 to output an electrical signal from the photoelectric conversion element 221 and, if any, electrical signals from the current /voltage conversion elements 222, the analog/digital conversion element 223, and the memory 224, and a photodetector control section 226 to control the photoelectric conversion element 221, the output section 225 and, if any, the current/voltage conversion element 222, the analog/digital conversion element 223, and the memory 224.

Thus, the electrical signal transmitted or received between the connection cable 200 and the physical quantity detector signal processing box 300 may be any one of a current signal provided after photoelectrically converting light modulated by the physical quantity detector 111, a voltage signal provided after subjecting the current signal to the current/voltage conversion, and a digital signal or a digital conversion value provided after subjecting the voltage signal to the analog/digital conversion.

Additionally, a method of transmitting or receiving (exchanging) the electrical signal may be either a parallel connection system or a serial connection system. A physical quantity to be detected, processing speed, and an influence of noise or the like are taken into consideration, and an output result from the physical quantity detector 111 can be transmitted to the physical quantity detector signal processing box 300 in an appropriate signal form.

FIG. 5 shows a connection example of the tubular system. In FIG. 5, the tubular apparatus 100 is depicted as an endoscope, and it is connected to a tubular apparatus image processing circuit 160 and a tubular apparatus illumination box 170 through a tubular system connector 150. The tubular apparatus image processing circuit 160 and the tubular apparatus illumination box 170 may be integrated. Further, the connection cable 200 is connected to the tubular system connector 150. Thus, the tubular apparatus 100 is connected to the physical quantity detector signal processing box 300 through the tubular system connector 150 and the connection cable 200.

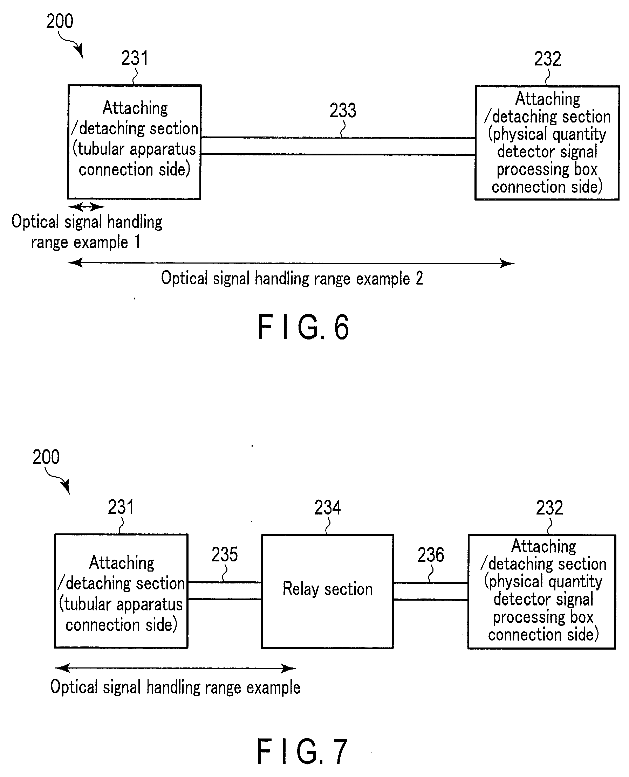

FIG. 6 shows a structural example of the connection cable 200. As shown in FIG. 6, the connector cable 200 includes an attaching/detaching section 231 configured to be attachable to or detachable from the tubular apparatus 100, an attaching/detaching section 232 configured to be attachable to or detachable from the physical quantity detector signal processing box 300, and a connection member 233 connecting the attaching/detaching section 231 to the attaching/detaching section 232.

The attaching/detaching section 231 is a connector section to be connected to the tubular apparatus 100. The attaching/detaching section 232 is a connector section to be connected to the physical quantity detector signal processing box 300. The connection member 233 may be formed of either a flexible member or a hard member, or it may be made of a composite material of the flexible member and the hard member.

The light entrance/exit section 213 is contained in the attaching/detaching section 231, and the electrical input/output section 215 is contained in the attaching/detaching section 232. The light source 211 may be contained in either the attaching/detaching section 231 or the attaching/detaching section 232. The photodetector 214 may be contained in either the attaching/detaching section 231 or the attaching/detaching section 232. If any, the optical distributing section 212 may be contained in, e.g., the attaching/detaching section 232 together with the light source 211 and the photodetector 214. Furthermore, the optical distributing section 212 may be contained in the attaching/detaching section 231 irrespective of contained positions of the light source 211 and the photodetector 214.

When both the light source 211 and the photodetector 214 are contained in the attaching/detaching section 231, an optical signal handling range ranges to the attaching/detaching section 231. Further, when at least one of the light source 211 and the photodetector 214 is contained in the attaching/detaching section 232, an optical signal handling range ranges to the attaching/detaching section 232.

The configuration that both the light source 211 and the photodetector 214 are contained in the attaching/detaching section 231 on the tubular apparatus connection side has an advantage in that the optical signal is converted into the electrical signal in the attaching/detaching section 231, so that handling of a cable near the attaching/detaching section 232 on the physical quantity detector signal processing box connection side is easier. Furthermore, the configuration that both the light source 211 and the photodetector 214 are contained in the attaching/detaching section 232 on the physical quantity detector signal processing box connection side is suitable for miniaturization of the attaching/detaching section 231 on the tubular apparatus connection side.

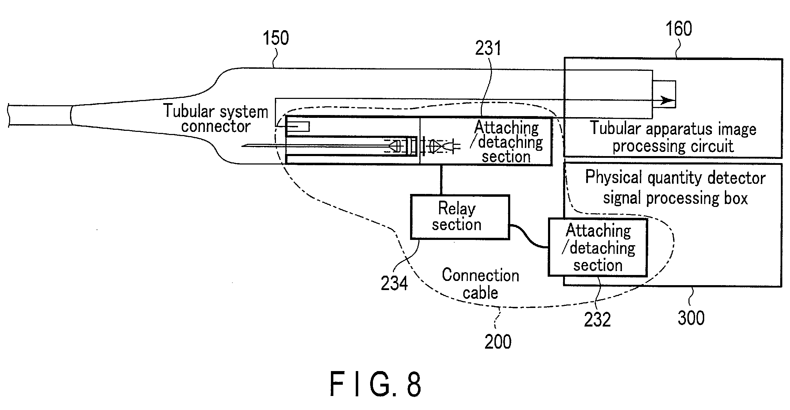

FIG. 7 shows another structural example of the connection cable 200, and FIG. 8 shows a connection example of the tubular system including this connection cable 200. As shown in FIG. 7, the connection cable 200 includes the attaching/detaching section 231 configured to be attachable to or detachable from the tubular apparatus 100, the attaching/detaching section 232 configured to be attachable to or detachable from the physical quantity detector signal processing box 300, a relay section 234 arranged between the attaching/detaching section 231 and the attaching/detaching section 232, a connection member 235 connecting the attaching/detaching section 231 to the relay section 234, and a connection member 236 connecting the relay section 234 to the attaching/detaching section 232.

The connection member 235 and the connection member 236 may be configured like the connection member 233.

The light entrance/exit section 213 is contained in the attaching/detaching section 231. The electrical input/output section 215 is contained in the attaching/detaching section 232. One of the light source 211 and the photodetector 214 is contained in the relay section 234. The other of the light source 211 and the photodetector 214 is contained in either the attaching/detaching section 231 or the relay section 234. If any, the optical distributing section 212 may be contained in, e.g., the relay section 234 together with the light source 211 and the photodetector 214. Moreover, the optical distributing section 212 may be contained in the attaching/detaching section 231 together with one of the light source 211 and the photodetector 214 or separately from the light source 211 and the photodetector 214.

In this structural example, in any case, an optical signal handling range ranges to the relay section 234.

The configuration that both the light source 211 and the photodetector 214 are contained in the relay section 234 is suitable for miniaturization of the attaching/detaching section 231 on the tubular apparatus connection side.

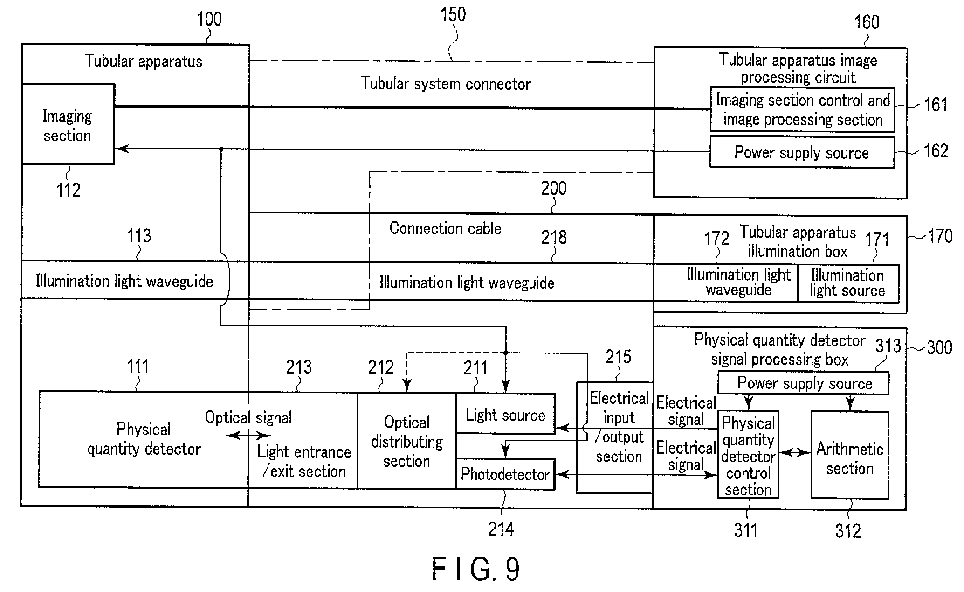

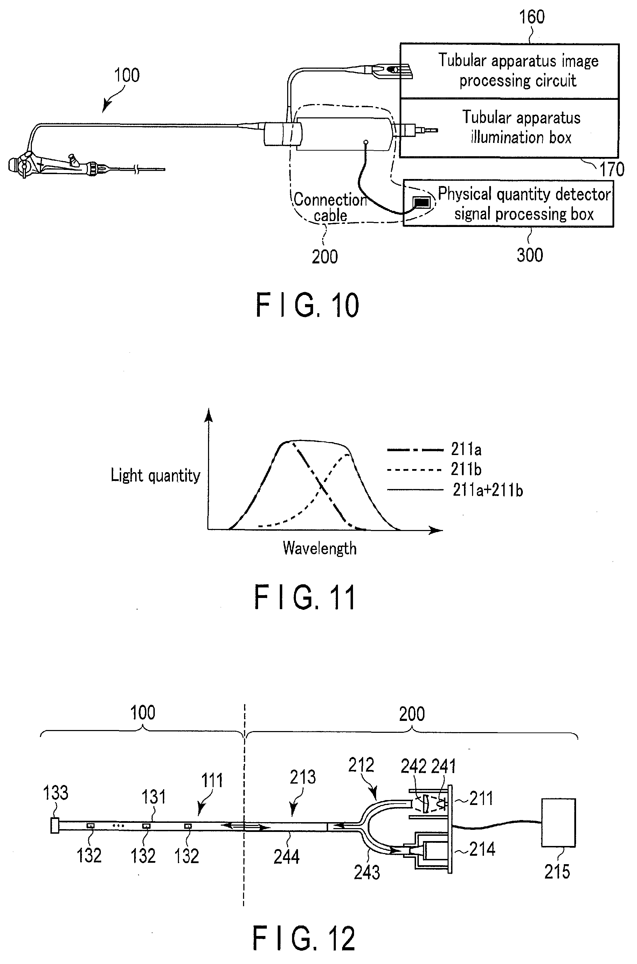

FIG. 9 shows still another example of the connection cable 200, and FIG. 10 shows a connection example of the tubular system including this connection cable 200. In these drawings, like reference numerals denote members equal to those shown in FIGS. 1 to 8 to omit a detailed description thereof. As shown in FIG. 10, the tubular apparatus 100 is an endoscope. As shown in FIG. 9, the tubular apparatus 100, i.e., the endoscope includes an imaging section 112 configured to photograph a specimen, and the imaging section 112 is arranged in an inserting section of the endoscope. The imaging section 112 is connected to an imaging section control and image processing section 161 and a power supply source 162 in the tubular apparatus image processing circuit 160 through the tubular system connector 150. The connection cable 200 includes an illumination light waveguide 218 to supply illumination light required for photographing by the endoscope, in addition to the light source 211, the photodetector 214, the light entrance/exit section 213, and the electrical input/output section 215 (and the optical distributing section 212). The illumination light waveguide 218 is optically connected with an illumination light waveguide 172 optically connected with an illumination light source 171 in a tubular apparatus illumination box 170 when it is connected to the tubular apparatus illumination box 170, and it is optically connected with an illumination light waveguide 113 in the tubular apparatus 100 when it is connected to the tubular apparatus 100. As can be understood from FIG. 10, it can be said that the connection cable 200 has a configuration that the tubular system connector 150 is partially included, in other words, a configuration that the attaching/detaching section 231 on the tubular apparatus connection side is integrated with the tubular system connector 150.

As described above, when the illumination light waveguide 218 is included in the connection cable 200, the connection cable 200 is readily arranged along a length direction of the tubular system connector 150. With this arrangement, an unnecessary protrusion is not present in a circumferential direction of the tubular system connector 150, and an easy-to-handle configuration can be provided. Moreover, with this configuration, since the illumination light waveguide 218 and the optical waveguide of the physical quantity detector 111 are arranged in parallel and they do not have to be bent with respect to the tubular system connector 150, the tubular system connector 150 can be easily miniaturized, and an easy-to-handle configuration can be provided. Additionally, the tubular apparatus 100 can be removed from the tubular apparatus illumination box 170 and the physical quantity detector signal processing box 300 only by disconnecting the tubular apparatus 100 from the tubular system connector 150.

The light source 211 may be constituted of at least one of a laser diode, an LED, and a lamp, or a fluorescent material to emit fluorescence upon receiving light from these members, or a combination of these members. Such a light source 211 is compactly mountable, and a size of the connection cable 200 can be reduced. A specific size can be close to a size of a cable connecting the tubular apparatus 100 to the tubular apparatus processing box. Further, since weight of such a light source 211 is light, the connection cable 200 can be configured with weight that does not trouble medical personnel. When return light exerts an influence on an output like a laser diode, the light source 211 may include an isolator (not shown) or the like.

Furthermore, the light source 211 may be configured by combining light-emitting elements. For example, FIG. 11 shows emission spectrums of the light source 211 constituted of two light-emitting elements 211a and 211b. In FIG. 11, an alternate long and short dash line indicates an emission spectrum of the light-emitting element 211a alone, a broken line indicates an emission spectrum of the light-emitting element 211b alone, and a solid line indicates an emission spectrum of the entire light source 211. As a result, it can supply light in a wavelength band over which one light-emitting element 211a or 211b alone does not extend. Moreover, when the light-emitting elements are changed over and used, it is possible to cope with the physical quantity detectors 111 or shift a detection time to perform detection.

FIG. 12 shows a connection example of the tubular apparatus 100 and the connection cable 200. As shown in FIG. 12, the light source 211 includes a light-emitting element 241, and a lens 242 to couple light from the light-emitting element 241 with the optical distributing section 212. The optical distributing section 212 includes an optical fiber coupler 243 using an optical fiber. The light entrance/exit section 213 includes an optical fiber 244. The optical fiber coupler 243 has three end portions, which are optically connected with the light source 211, the photodetector 214, and the optical fiber 244, respectively.

The physical quantity detector 111 includes an optical fiber 131 as an optical waveguide, the detection object section 132 provided to the optical fiber 131, and a reflection member 133 provided at a distal end of the optical fiber 131. The optical fiber 131 is arranged along an inserting section of the tubular apparatus 100. The detection object section 132 has a function of modulating light propagated in the optical fiber 131 in dependence on a physical quantity. The reflection member 133 is, e.g., a mirror formed by vapor-depositing aluminum or the like on the optical fiber. The reflection member 133 functions to return light that has been supplied from the light source 211 and reached an end of the optical fiber 131 via the detection object section 132 to the photodetector 214 side.

Once connecting the tubular apparatus 100 with the connection cable 200, the optical fiber 131 is optically coupled with the optical fiber coupler 243. That is, the light entrance/exit section 213 is connected with the physical quantity detector 111 through the connection of the optical fibers. The connection between the fibers is advantageous to configuring the light entrance/exit section 213 and the physical quantity detector 111 with reduced weight.

Since the optical distributing section 212 is constituted of the optical fiber coupler 243, the optical distributing section 212 can be miniaturized.

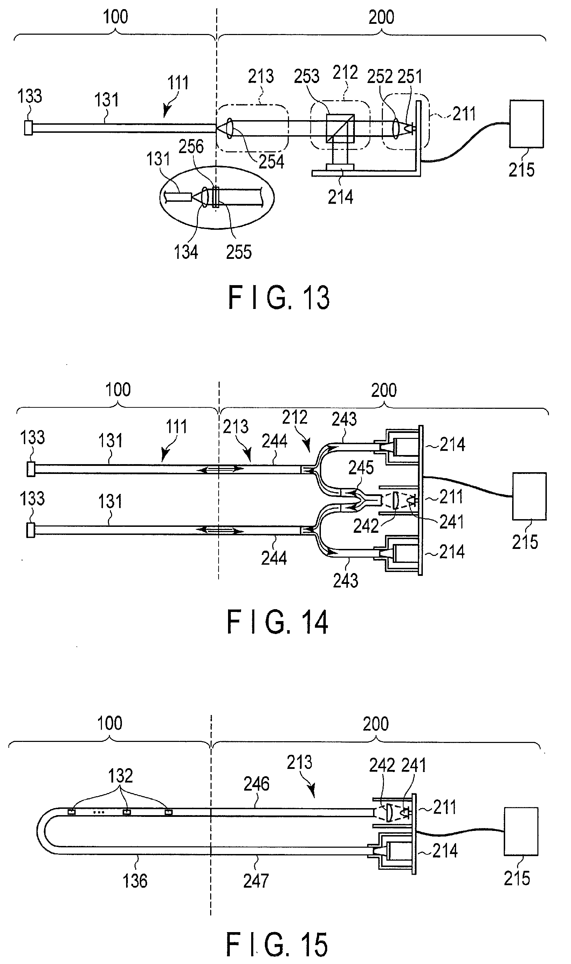

FIG. 13 shows another connection example of the tubular apparatus 100 and the connection cable 200. Like reference numerals denote members equal to those shown in FIG. 12 to omit a detailed description thereof. As shown in FIG. 13, the light source 211 includes a light-emitting element 251, and a lens 252 to change light from the light-emitting element 251 into parallel light. The optical distributing section 212 includes a beam splitter 253. The light distributing section 212 may have a half mirror in place of the beam splitter 253. The optical entrance/exit section 213 includes a lens 254 that is a converging optical system to converge the parallel light from the optical distributing section 212, coupling it with the optical fiber 131. In this connection example, once connecting the tubular apparatus 100 with the connection cable 200, the light entrance/exit section 213 is connected to the physical quantity detector 111 through the converging optical system. In the connection cable 200, light does not have to be caused to enter the optical fiber.

Moreover, as shown in an ellipse in FIG. 13, instead that the light entrance/exit section 213 includes the lens 254, the light entrance/exit section 213 may include a cover glass 255 and the physical quantity detector 111 may include a cover glass 135 and a lens 134 that is a converging optical system to converge the parallel light from the light entrance/exit section 213, coupling it with the optical fiber 131. In this connection example, once connecting the tubular apparatus 100 and the connection cable 200, the light entrance/exit section 213 is connected with the physical quantity detector 111 through the parallel light connection. The cover glass 255 of the light entrance/exit section 213 and the cover glass 135 of the physical quantity detector 111 function as dust removers. Even if dust is attached to these members, since the optical connection is not completely interrupted, the optical connection can be easily carried out.

FIG 14 shows still another example of the tubular apparatus 100 and the connection cable 200. Like reference numerals denote members equal to those shown in FIG. 12 to omit a detailed description thereof. As shown in FIG. 14, the connection cable 200 includes one light source 21 and two photodetectors 214. The light entrance/exit section 213 includes two optical fibers 244. The optical distributing section 212 includes three optical fiber couplers 243 and 245. One end portion of the optical fiber coupler 245 is connected to the light source 211, and other two end portions of the same are connected to end portions of the respective optical fiber couplers 243 on one side respectively. Another end portion of each optical fiber coupler 243 is connected to the photodetector 214, and the remaining one end portion of the same is connected to the optical fiber 244. The physical quantity detector 111 includes two optical fibers 131 and two reflection members 133. Once connecting the tubular apparatus 100 with the connection cable 200, the optical fiber 131 is optically coupled with the optical fiber 244. That is, the light entrance/exit section 213 is connected with the physical quantity detector 111 through the connection of the optical fibers.

Since the optical distributing section 212 includes the three optical fiber couplers 243 and 245, it is possible to cope with the physical quantity detector 111 having the two optical fibers 131. When the optical distributing section 212 is configured to include more optical fiber couplers, it is possible to cope with a configuration that the physical quantity detector 111 includes more optical fibers 131.

FIG. 15 shows yet another connection example of the tubular apparatus 100 and the connection cable 200. Like reference numerals denote members equal to those shown in FIG. 12 to omit a detailed description thereof. As shown in FIG. 15, the connection cable 200 includes no optical distributing section, and the light entrance/exit section 213 includes an optical fiber 246 connected with the light source 211, and an optical fiber 247 connected to the photodetector 214. The physical quantity detector 111 includes an optical fiber 136 extending in a loop-like shape. The detection object section 132 is provided to the optical fiber 136. Once connecting the tubular apparatus 100 and the connection cable 200, one end portion of the optical fiber 136 is optically coupled with the optical fiber 246, and the other end portion is optically coupled with the optical fiber 247. That is, the light entrance/exit section 213 is connected to the physical quantity detector 111 through the connection between the optical fibers.

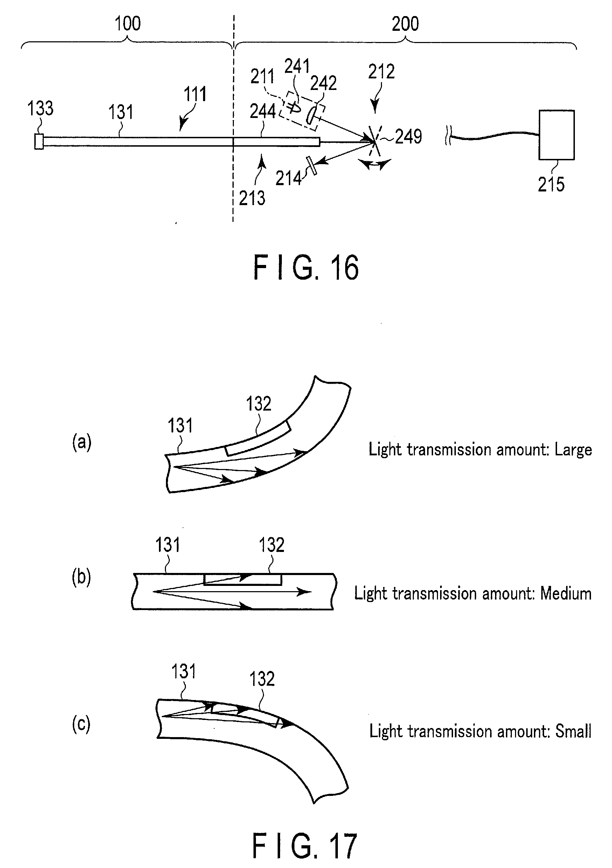

FIG. 16 shows still yet another connection example of the tubular apparatus 100 and the connection cable 200. Like reference numerals denote members equal to those shown in FIG. 12 to omit a detailed description. As shown in FIG. 16, the optical distributing section 212 includes an MEMS mirror 249 that functions as a light deflection element. The light source 211 may emit light having a wavelength multiplexing component, and the optical distributing section 212 may include a spectroscopic element.

In the tubular system shown in FIG. 12 to FIG. 16, the physical quantity detector 111 in the tubular apparatus 100 as well as the light source 211, the photodetector 214, the light entrance/exit section 213 and, if any, the optical distributing section 212 in the connection cable 200 constitute a fiber sensor to detect physical quantities. For example, the fiber sensor is a shape sensor to detect a change in shape of the inserting section of the tubular apparatus 100 based on a change in shape applied to the optical waveguide with a change in shape of the inserting section of the tubular apparatus 100. This fiber sensor, which is the shape sensor, is a sensor to acquire a direction and a magnitude of curving of the detection object section 132 provided in the optical fiber 131 by detecting a relationship in characteristics between light entering the optical fiber 131 and light exiting from the optical fiber 131. When the fiber sensor is used, it is possible to suppress an area occupied by the optical waveguide, i.e., the optical fiber 131 along a radial direction of the inserting section of the tubular apparatus 100.

Since the fiber sensor detects physical quantities based on characteristics of entrance/exit light, it is possible that the optical fibers 131 and 136 each having the detection object section 132 formed thereto alone are arranged in the tubular apparatus 100, and the light source 211 and the photodetector 214 are mounted in the connection cable 200. Consequently, a connection point for light is provided at only one position between the tubular apparatus 100 and the connection cable 200, and the easy-to-handle tubular system having less signal loss can be provided. Further, since the connection cable 200 containing the light source 211 and the photodetector 214 does not have to be configured with resistance to sterilization using a high-temperature/high-voltage/corrosive gas, miniaturization is possible at low costs. Furthermore, since there are not many components arranged in the tubular apparatus 100, the tubular apparatus 100 can be provided at low costs. The optical fiber 131 provided with the detection object section 132 is mounted in the flexible inserting section of the tubular apparatus 100. When the inserting section curves, the optical fiber 131 curves in accordance with this curving, and a part of light propagated through the optical fiber 131 exits (leaks) to the outside through the detection object section 132 with this curving. That is, the detection object section 132 is provided on one side surface of the optical fiber 131, and causes a part of the propagated light to exit to the outside in accordance with the curving of the optical fiber 131. That is, the detection object section 132 changes optical characteristics, a light transmission amount of the optical fiber 131.

FIG. 17 is schematic views of light transmission amounts according to curving of the optical fiber 131. In the drawing, an upper section (a) shows a state when the optical fiber 131 curves to a side where the detection object section 132 is provided, a middle section (b) shows a state when the optical fiber 131 does not curve, and a lower section (c) shows a state when the optical fiber 131 curves to a side opposite to the side where the detection object section 132 is provided. As shown in FIG. 17, when the optical fiber 131 curves to the side where the detection object section 132 is provided, since an amount of light that enters the detection object section 132 increases, a light transmission amount of the optical fiber 131 becomes larger than that when the optical fiber 131 does not curve. Furthermore, the light transmission amount increases as the curving becomes greater. Contrarily, when the optical fiber 131 curves to the side opposite to the side where the detection object section 132 is provided, since the amount of light that enters the detection object section 132 decreases, the light transmission amount of the optical fiber 131 is reduced to be smaller than that when the optical fiber 131 does not curve. Moreover, the light transmission amount decreases as the curving becomes greater.

In this manner, when the optical fiber 131 curves with the curving of the flexible inserting section of the tubular apparatus 100, the light transmission amount of the optical fiber 131 changes in accordance with a direction and a magnitude of this curving. An optical signal including information of this change in light transmission amount is converted into an electrical signal by the photodetector 214 and transmitted to the arithmetic section 312, and a bent shape, i.e., a direction and a magnitude of bending of the inserting section in an actually bent portion are calculated.

Here, the example of the light transmission amount has been described as the optical characteristics to change, but the present invention is not restricted thereto, and a state of light, e.g., a spectrum or polarization may be adopted. In this case, the fiber sensor could detect a state of light, e.g., a spectrum or polarization.

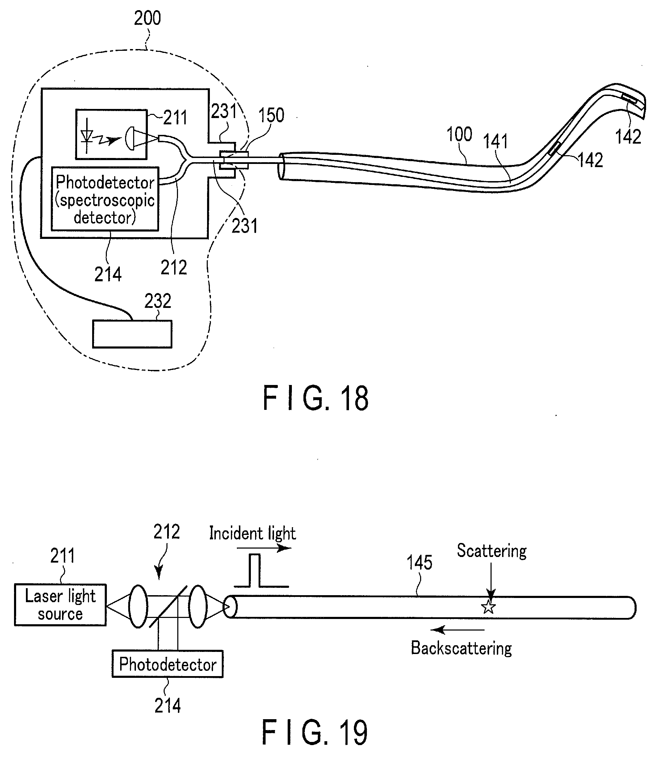

FIG. 18 shows another structural example of the fiber sensor. This fiber sensor is a temperature sensor capable of detecting temperatures. The light source 211 is configured to emit light in a broadband. The photodetector 214 has a function of a spectroscopic detector. An optical fiber 141 arranged in the inserting section of the tubular apparatus 100 has a core on which FBGs 142 is formed. The portions of the optical fiber 141 where the FBGs 142 are formed are fixed at positions of the inserting section of the tubular apparatus 100 to which distortion is not applied, or they are configured not to receive the distortion and then fixed to the inserting section of the tubular apparatus 100. The FBGs 142 reflect light having a specific wavelength, and allow light having remaining wavelengths to transmit therethrough. The two FBGs 142 in the drawing are configured to reflect light having different specific wavelengths.

When light in a broadband emitted from the light source 211 enters the optical fiber 141, light having a specific wavelength is reflected and the remaining light are transmitted by the FBGs 142. A specific wavelength λB to be reflected is called a Bragg wavelength, and it can be represented as λB=2·n·d by using an effective refractive index n of the core and an interval d of a diffraction grating. The grating interval and the refractive index vary in dependence on distortion or temperatures. Since the distortion is not applied to the portions of the optical fiber 141 where the FBGs 142 are formed, the grating interval and the refractive index vary in dependence on temperatures. When the grating interval and the refractive index vary, the Bragg wavelength shifts, and hence the wavelength of light reflected by the FBGs 142 changes. The wavelength of light reflected by the FBGs 142 is detected by the photodetector 214, and hence a change in temperature is detected.

FIG. 19 shows another structural example of the temperature sensor. This temperature sensor uses scattered light in an optical fiber 145. The light source 211 emits pulsed light. This pulsed light enters the optical fiber 145 arranged in the inserting section of the tubular apparatus through the optical distributing section 212. A part of light propagated in the optical fiber 145 is scattered in the optical fiber 145. As scattering, there are Brillouin scattering, Raman scattering, and the like. Backscattered light is propagated in the optical fiber 145 in a direction opposite to the propagating direction of the incident light, and detected by the photodetector 214 through the optical distributing section 212. A frequency shift amount of the scattered light (a long wavelength side: Stokes light, a short wavelength side: anti-Stokes light) to the incident light is dependent on temperatures. The temperatures can be acquired by calculating the frequency shift amount between the incident light from the light source 211 and the scattered light detected by the photodetector 214. A scattering location is identified based on a return light time of the scattered light.

Although the embodiments according to the present invention have been described with reference to the drawings, the present invention is not restricted to these embodiments, and it may be modified or changed in many ways without departing from the scope of its gist. The various modifications or changes include embodiments provided by appropriately combining the foregoing embodiments.

## Claims

1. A tubular system comprising a tubular apparatus comprising a flexible inserting section and a physical quantity detector configured to detect a physical quantity, a physical quantity detector signal processing box to process a signal from the physical quantity detector, and a detachable connection cable to connect the tubular apparatus with the physical quantity detector signal processing box, **characterized in that**
the physical quantity detector comprises an optical waveguide arranged in the inserting section; the connection cable comprises a light source to supply light to the optical waveguide of the physical quantity detector, a photodetector to detect light modulated by the physical quantity detector, a light entrance/exit section to supply or receive an optical signal to or from the physical quantity detector, and an electrical input/output section to supply or receive an electrical signal to or from the physical quantity detector signal processing box; and the physical quantity detector, the light source, the photodetector, and the light entrance/exit section constitute a sensor to detect the physical quantity.

2. The system according to claim 1, **characterized in that** the connection cable comprises a first attaching/detaching section configured to be attachable to or detachable from the tubular apparatus, a second attaching/detaching section configured to be attachable to or detachable from the physical quantity detector signal processing box, and a connection member connecting the first attaching/detaching section with the second attaching/detaching section, the light entrance/exit section is contained in the first attaching/detaching section, the electrical input/output section is contained in the second attaching/detaching section, the light source is contained in either the first attaching/detaching section or the second attaching/detaching section, and the photodetector is contained in either the first attaching/detaching section or the second attaching/detaching section.

3. The system according to claim 2, **characterized in that** the connection cable further comprises an optical distributing section defining flows of light between the light entrance/exit section, the light source, and the photodetector, and the optical distributing section is contained in the second attaching/detaching section together with the light source and the photodetector or contained in the first attaching/detaching section.

4. The system according to claim 1, **characterized in that** the connection cable comprises a first attaching/detaching section configured to be attachable to or detachable from the tubular apparatus, a second attaching/detaching section configured to be attachable to or detachable from the physical quantity detector signal processing box, a relay section arranged between the first attaching/detaching section and the second attaching/detaching section, a first connection member connecting the first attaching/detaching section with the relay section, and a second connection member connecting the relay section with the second attaching/detaching section, the light entrance/exit section is contained in the first attaching/detaching section, the electrical input/output section is contained in the second attaching/detaching section, one of the light source and the photodetector is contained in the relay section, and the other of the light source and the photodetector is contained in either the first attaching/detaching section or the relay section.

5. The system according to claim 4, **characterized in that** the connection cable further comprises an optical distributing section defining flows of light between the light entrance/exit section, the light source, and the photodetector, and the optical distributing section is contained in the relay section together with the light source and the photodetector or contained in the first attaching/detaching section.

6. The system according to any one of claims 1 to 5, **characterized in that** the tubular apparatus is an endoscope, and the connection cable further comprises an illumination light waveguide to supply illumination light required for photographing by the endoscope.

7. The system according to any one of claims 1 to 5, **characterized in that** the light source comprises at least one of a laser diode, an LED, and a lamp, or a fluorescent material to emit fluorescence upon receiving light from these members, or a combination of these members.

8. The system according to any one of claims 1 to 5, **characterized in that** the electrical signal supplied or received between the connection cable and the physical quantity detector signal processing box is one of a current signal provided after photoelectrically converting light modulated by the physical quantity detector, a voltage signal provided after performing current/voltage conversion to the current signal, and a digital signal provided after performing analog/digital conversion to the voltage signal.

9. The system according to any one of claims 1 to 5, **characterized in that** the light entrance/exit section is connected to the physical quantity detector through one of connection between optical fibers, a converging optical system, and parallel light connection.

10. The system according to any one of claims 1 to 5, **characterized in that** the sensor is a shape sensor to detect a change in shape of the inserting section based on a change in shape applied to the optical waveguide with the change in shape of the inserting section of the tubular apparatus.

11. The system according to claim 3 or 5, **characterized in that** the optical distributing section comprises at least one of an optical fiber coupler using optical fibers, a beam splitter, a half mirror, an MEMS mirror, and a spectroscopic element.
